(19) **Europäisches Patentamt**

**European Patent Office**

**Office européen des brevets**

(11) **EP 1 129 991 A1**

(12) **EUROPÄISCHE PATENTANMELDUNG**

(43) Veröffentlichungstag:
05.09.2001 Patentblatt 2001/36

(51) Int Cl.⁷: **C01B 37/00**, B01J 29/04,
C07D 301/12

(21) Anmeldenummer: 00104324.9

(22) Anmeldetag: 02.03.2000

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(71) Anmelder: **Degussa AG**
**40474 Düsseldorf (DE)**

(72) Erfinder:
• **Hasenzahl, Steffen**
**63477 Maintal (DE)**

• **Heyne, Klaus**
**63486 Bruchkobel (DE)**
• **Kneitel, Dieter**
**63517 Rodenbach (DE)**

(74) Vertreter:
**Sternagel, Fleischer, Godemeyer & Partner**
**Patentanwälte**
**Braunsberger Feld 29**
**51429 Bergisch Gladbach (DE)**

(54) **Verfahren zur Herstellung eines titanhaltigen Zeolithen**

(57)    Die vorliegende Erfindung betrifft ein Verfahren zur Herstellung eines titanhaltigen Zeolithen, wobei ein Synthesegel durch Zusammengeben und Hydrolisieren einer hydrolisierbaren Siliciumverbindung, einer hydrolisierbaren Titanverbindung und einer basischen quartären Ammoniumverbindung in wäßrigem Medium in Mengen, so daß sich bezogen auf die Ausgangsverbindungen ein molares Verhältnis von Si/Ti von größer gleich 30 und von N/Si von 0,12 bis kleiner 0,20 ergibt, ausgebildet wird und anschließend das Synthesegels bei einer Temperatur von 150°C bis 220°C für eine Zeitdauer von weniger als 3 Tage kristallisiert wird sowie einen nach diesem Verfahren erhältlichen titanhaltigen Zeolithen.

**Beschreibung**

Die vorliegende Erfindung betrifft ein Verfahren zur Herstellung eines titanhaltigen Zeolithen, einen durch dieses Verfahren erhältlichen titanhaltigen Zeolithen, ein Verfahren zur Epoxidierung von Olefinen in Gegenwart eines so hergestellten titanhaltigen Zeolithen sowie auch die Verwendung eines solchen Zeolithen als Katalysator für die Epoxidierung von Olefinen.

Aus US-A-4,410,501 ist ein Verfahren zur Herstellung von Titansilicalit wie auch die Verwendung des Titansilicalits als Katalysator in einer Reihe von Reaktionen, u. a. auch Oxidationsreaktionen bekannt. Hierin sind zwei verschiedene Verfahrensweisen beschrieben. Zum einen die Ausbildung eines Synthesegels ausgehend von einer hydrolysierbaren Siliciumverbindung wie z. B. Tetraethylorthosilikat oder alternativ die Verwendung eines kolloidalen Siliciumdioxids als Siliciumquelle. Die erste Variante ist in Beispiel 1 der US-A-4,410,501 beschrieben. Hierin wird Tetraethylorthosilikat und Tetra-n-propylammoniumhydroxid (TPAOH) in einem molaren Verhältnis von 0,45 eingesetzt, was auch dem bevorzugten Bereich für das molare Verhältnis von Ammoniumverbindung zu Siliciumverbindung wie im allgemeinen Teil der Beschreibung angegeben, entspricht. Beiden Verfahrensvarianten gemeinsam ist nach der Beschreibung in US-A-4,410,501 eine lange Reaktionsdauer für den Hydrothermal schritt von mindestens sechs Tagen.

In EP-A 0 838 431 ist ein Verfahren zur Herstellung von titanhaltigen Zeolithen ausgehend von Tetraalkylorthosilikat und Tetraalkylorthotitanat beschrieben, bei dem es wesentlich darauf ankommt, daß die Reaktionsmischung ohne Abdestillation der bei der Hydrolyse entstehenden Alkohole in einem Autoklaven unter Hydrothermalbedingungen umgesetzt wird.

Viele Forschergruppen in Industrie und Hochschule haben versucht, ausgehend von der Lehre von US-A-4,410,501 die Synthese von Titansilicalit sowohl bezüglich der Aktivität des resultierenden Katalysators als auch bezüglich der Verfahrenseffizienz, d. h. Verringerung der Dauer für den Hydrothermalschritt, Verbesserung der Ausbeute usw. zu optimieren.

So haben A. J. H. P. van der Pol und J. H. C. van Hooff in "Applied Catalysis A: General, 92 (1992) 93-111" die Auswirkung der $SiO_2$-Quelle, der Kristallisationsdauer, den Kristallisationsbedingungen sowie das Verhältnis Ammoniumverbindung/Silicium und Silicium/Titan auf die Titansilicalitsynthese sowie auf die Aktivität des resultierenden Katalysators untersucht. Bei diesen Experimenten wurde sowohl das Verfahren, ausgehend von Tetraethylorthosilikat als auch die Variante, ausgehend von einem kollodialen Siliciumdioxid untersucht. Ein Vergleich dieser beiden Methoden ergibt bereits, daß für das Esterverfahren, d. h. Verwendung von Tetraethylorthosilikat ein höheres Verhältnis von Ammoniumverbindung zu Si zu verwenden ist. Dies entspricht insoweit der Lehre der US-A-4,410,501, wo für die Orthosilikatroute ebenfalls ein deutlich höheres Verhältnis von Ammonium zu Silicium eingesetzt wird. Weiterhin ergibt sich, daß unabhängig von der eingesetzten Methode Kristallisationszeiten von zwei Tagen ausreichend sind für die Herstellung des Katalysators. Weiterhin lehrt diese Veröffentlichung, daß ein Titansilicalit, der unter Verwendung von Orthosilikat hergestellt wurde, eine deutlich höhere katalytische Aktivität zeigt als Katalysatoren, die unter Verwendung von kollodialem Siliciumdioxid hergestellt wurde. Ein wesentlicher Teil der oben angegebenen Veröffentlichung beschäftigt sich mit der Frage, wie die Reaktionsbedingungen, insbesondere die Verhältnisse der Ausgangsverbindungen gewählt werden sollen, um möglichst kleine Primärkristallite des Katalysators herzustellen. Insbesondere wurde der Einfluß des Verhältnisses Ammoniumverbindung/Silicium in den Ausgangsverbindungen auf die Kristallitgröße untersucht. Dabei wurde festgestellt, daß das molare Verhältnis Ammoniumverbindung/Silicium mindestens 0,22 sein sollte, besser im Bereich zwischen 0,3 und 0,35 liegen sollte, um die gewünschte Primärkristallitgröße zu erhalten. Dahingegen hat das Verhältnis von Si zu Ti nahezu keinen Einfluß auf die Kristallitgröße des Titansilicalits.

Diese Ergebnisse wurden durch eine weitere Untersuchung von van der Pol, Verduyn und van Hooff in "Applied Catalysis A: General, 92 (1992) 113-130" bestätigt. Hierin wurde die Aktivität verschiedener Titansilicalitkatalysatoren bei der Hydroxylierung von Phenol untersucht. Es wurde festgestellt, daß die Aktivität eine Funktion der Kristallitgröße ist und mit abnehmender Kristallitgröße zunimmt. Die Kristallitgröße wiederum hängt von dem Verhältnis Ammonium/Silicium ab, wobei ein Verhältnis von 0,35 zu einer Kristallitgröße von 0,2 µm führen wird und die größte Aktivität zeigt. Höhere Verhältnisse von Ammonium zu Silicium von 0,53 bzw. 0,78 führen zu deutlich größeren Kristalliten und geringeren Aktivitäten. Ein niedrigeres Verhältnis von 0,22 führt ebenfalls zu größeren Kristalliten und ebenfalls zu einer Abnahme der Aktivität.

Weiterhin zeigen diese Untersuchungen, daß in dem untersuchten Bereich des Verhältnisses Ammoniumverbindung/Silicium der Anteil an eingebautem Titan bei konstantem Silicium- zu Titanverhältnis in der Ausgangszusammensetzung nahezu konstant bleibt.

Diese Untersuchungen führten zu der Entwicklung einer Standardsynthese für einen Titansilicalitkatalysator, der mit EUROTS-1-Katalysator bezeichnet wurde und dessen Synthese in J. A. Martens et al. in "Applied Catalysis A: General, 99 (1993) 71-84" veröffentlicht ist. Bei der Synthese von EUROTS-1 wird Tetraethylorthosilikat, Tetraethylorthotitanat und Tetrapropylammoniumhydroxid in einem molaren Verhältnis von Si zu Ti gleich 35, Ammonium zu Si gleich 0,36 und $H_2O$ zu $SiO_2$ gleich 28,2 zusammengegeben, hydrolysiert und vier Tage lang bei 175°C kristallisiert.

Trotz dieser sehr weitgehenden und tiefgehenden Auseinandersetzung mit den Parametern, die die Synthese und Aktivität von Titansilicalitkatalysatoren beeinflussen und der oben beschriebenen Entwicklung eines hochaktiven Standardkatalysators besteht dennoch nach wie vor ein Bedürfnis in der Industrie, die Synthese von Titansilicalitkatalysatoren effektiv und kostengünstiger zu gestalten und, falls möglich, dabei noch die Aktivität, insbesondere für die Epoxidierung von Olefinen des Katalysators zu steigern.

Aufgabe der vorliegenden Erfindung ist es daher, ein effizienteres und kostengünstigeres Verfahren zur Herstellung von titanhaltigen Zeolithen bereitzustellen, ohne daß dadurch die katalytische Aktivität des resultierenden Zeolithen beeinträchtigt wird. Weiterhin ist es eine Aufgabe, einen titanhaltigen Zeolithen mit einer verbesserten Aktivität für die Epoxidierung von Olefinen mit Wasserstoffperoxid bereitzustellen.

Diese Aufgabe wird durch ein Verfahren zur Herstellung eines titanhaltigen Zeolithen, wobei ein Synthesegel durch Zusammengeben und Hydrolisieren einer hydrolisierbaren Siliciumverbindung, einer hydrolisierbaren Titanverbindung und einer basischen quartären Ammoniumverbindung in wäßrigem Medium in Mengen, so daß sich bezogen auf die Ausgangsverbindungen ein molares Verhältnis von Si/Ti von größer gleich 30 und von N/Si von 0,12 bis kleiner 0,20 ergibt, ausgebildet wird und anschließend das Synthesegel bei einer Temperatur von 150°C bis 220°C für eine Zeitdauer von weniger als 3 Tage kristallisiert wird sowie durch einen nach einem solchem Verfahren erhältlichen titanhaltigen Zeolithen gelöst.

Entsprechend der vorliegenden Erfindung werden zunächst eine hydrolysierbare Siliciumverbindung und eine hydrolysierbare Titanverbindung mit einer basischen quartären Ammoniumverbindung in Gegenwart von Wasser hydrolysiert. Dabei kommt es wesentlich darauf an, daß die Ausgangsverbindungen in bestimmten molaren Verhältnissen miteinander umgesetzt werden.

Dabei kann das molare Verhältnis von Si zu Ti der Ausgangsverbindungen in weiten Bereichen variiert werden, solange es $\geq$ 30 ist. Im Gegensatz dazu muß das molare Verhältnis von N zu Si der Ausgangsverbindung im engen Bereich von 0,12 bis < 0,20 gehalten werden.

Es wurde überraschenderweise festgestellt, daß in diesem Bereich des molaren Verhältnisses von N zu Si zwar entsprechend der Lehren von A. van der Pol und J. H. C. van Hooff (a.a.o.) die Kristallitgröße im Vergleich zu dem als optimal betrachteten Verhältnis von 0,35 deutlich zunimmt, aber dennoch in diesem engen Bereich entgegen den von van der Pol und van Hooff aufgestellten Lehren die Katalysatoraktivität in einer Propylenepoxidierungsreaktion mit Wasserstoffperoxid zunimmt. Ohne sich an eine bestimmte Theorie gebunden zu fühlen, wird angenommen, daß dieser Effekt dadurch erzielt wird, daß in dem angegebenen Bereich für das molare Verhältniss von N zu Si bei gleichbleibendem Si- zu Ti-Verhältnis proportional mehr Titan in die Kristallstruktur des Zeolithen eingebaut wird. Die dadurch verbundene Aktivitätssteigerung überkompensiert den Verlust der Aktivität durch Zunahme der Primärkristallitgröße. Dieses Ergebnis ist um so überraschender, da van der Pol und van Hooff (a.a.o.) gezeigt haben, daß bei einem N- zu Si-Verhältnis von 0,22 und größer bei gleichbleibendem Si- zu Ti-Verhältnis der Ausgangsverbindungen die Menge an in den Zeolithen eingebautes Titan konstant bleibt und im wesentlichen unabhängig vom N- zu Si-Verhältnis ist.

Besonders gute Ergebnisse bzgl. der Katalysatoraktivität in Epoxidierungsreaktionen werden bei einem molaren Verhältnis der Ausgangsverbindungen von N zu Si im Bereich von 0,12 bis 0,17 erzielt.

Als hydrolysierbare Silicium- bzw. Titanverbindungen eignen sich insbesondere für das erfindungsgemäße Verfahren die Tetraalkylorthosilikate bzw. Tetraalkylorthotitanate, wobei Alkyl vorzugsweise ausgewählt ist aus der Gruppe bestehend aus Methyl, Ethyl, Propyl oder Butyl. Die am meisten bevorzugten Ausgangsverbindungen sind Tetraethylorthosilikat und Tetraethylorthotitanat.

Bei der quartären Ammoniumverbindung handelt es sich um eine Templatverbindung, die durch Aufnahme in das Kristallgitter des Produkts während der Kristallisation die Kristallstruktur bestimmt. Bevorzugt werden Tetraalkylammoniumverbindungen wie Tetraalkylammoniumhydroxid, insbesondere Tetra-n-propylammoniumhydroxid zur Herstellung von Titansilicalit-1 (MFI-Struktur), Tetra-n-butylammoniumhydroxid zur Herstellung von Titansilicalit-2 (MEL-Struktur) und Tetraethylammoniumhydroxid zur Herstellung von Titan-β-Zeolith (BEA-Kristallstruktur) eingesetzt. Die quartäre Ammoniumverbindung wird vorzugsweise als wäßrige Lösung eingesetzt.

Der für die Synthese erforderliche pH-Wert des Synthesesols von > 9, bevorzugt > 11, stellt sich durch die basisch reagierende quartäre Ammoniumverbindung ein.

Die Temperatur, bei der das Synthesesol hergestellt wird, kann in weiten Grenzen gewählt werden, bevorzugt ist allerdings das Gemisch aus hydrolysierbarer Siliciumverbindung und hydrolysierbarer Titanverbindung auf eine Temperatur im Bereich von 0°C bis 10°C, vorzugsweise 0°C bis 5°C, abzukühlen und dann die basische quartäre Ammoniumverbindung in wäßriger Lösung, die auf die gleiche Temperatur abgekühlt ist, zuzutropfen. Gemäß einer alternativen Ausführungsform wird Tetraethylorthosilikat und Tetraethylorthotitanat vor der Hydrolyse auf 35°C erwärmt und bei dieser Temperatur 30 Minuten lang gerührt, um eine Komplexbildung zwischen den beiden Produkten zu erreichen (Vorkondensation). Diese Vorkondensation hat aber keinen merklichen Einfluß auf die katalytischen Eigenschaften des Endprodukts.

In einer weiteren Ausführungsform der vorliegenden Erfindung wird bei der Verwendung von Tetraalkylorthosilikaten und Tetraalkylorthotitanaten als Silicium- bzw. Titanquelle der bei der Hydrolyse entstehende Alkohol als Wasser-Azeotrop abdestilliert. In manchen Fällen kann es dann ratsam sein, das durch Abdestillieren entnommene Volumen an Alkohol-Wasser-Azeotrop zumindest teilweise in der Reaktionsmischung durch Wasser zu ersetzen, um die Ausbildung eines festen Gels bzw. Wandablagerung während der Kristallisation zu vermeiden.

Das Synthesegel wird anschließend, ggf. nach einer zusätzlichen Reifezeit bei einer Temperatur von 150°C bis 220°C, vorzugsweise 170°C bis 190°C unter autogenem Druck kristallisiert (Hydrothermalsynthese). Unter den vorgegebenen Bedingungen des erfindungsgemäßen Verfahrens beträgt die Kristallisationszeit weniger als 3 Tage, vorzugsweise weniger als 24 Stunden, besonders bevorzugt höchstens 12 Stunden.

Gemäß einer bevorzugten Ausführungsform des erfindungsgemäßen Verfahrens werden die Mengen der Ausgangsverbindungen so gewählt, daß sich bezogen auf die Ausgangsverbindungen ein molares Verhältnis von $H_2O$ zu Si im Bereich von 10 bis 20, vorzugsweise 12 bis 17 einstellt. Dabei ist es besonders vorteilhaft, wenn das molare Verhältnis von $H_2O$ zu Si im Synthesegel vor der Hydrothermalsynthese, d.h. nach dem eventuell zumindest teilweisen Ersatz des gegebenenfalls nach der Hydrolyse abdestillierten Wasser-Alkohol-Azeotrops durch Wasser in einem Bereich von 15 bis 42 vorzugsweise 15 bis 35 eingestellt wird. Es wurde überraschenderweise festgestellt, daß trotz dieser sehr geringen Wassermenge die Prozeßführung und die katalytische Aktivität des resultierenden Produkts nicht beeinträchtigt wird. Im Gegenteil es wurde gefunden, daß ein molares Verhältnis von Wasser zu Si im oben angegebenen Bereich in Kombination mit dem erfindungsgemäßen molaren Verhältnis von Ammoniumverbindung zu Si in der Ausgangszusammensetzung zu einem aktiveren Katalysator führt. Ein Grund hierfür könnte in der hohen Konzentration an quartärer Ammoniumverbindung im Synthesegel trotz deutlich verringerter Mengen an Ammoniumverbindung in der Ausgangsverbindung aufgrund der ebenfalls geringen Wassermengen zu sehen sein.

Ein weiterer Vorteil der geringen Wassermenge liegt in der deutlich im Vergleich zum Stand der Technik gesteigerten Ausbeute an titanhaltigem Zeolith pro kg Synthesegel, was das Gesamtverfahren effektiver und wirtschaftlicher macht.

Die nach dem Hydrothermalschritt entstandenen Kristalle haben eine Primärkristallitgröße im Bereich zwischen 0,2 bis 2,0 μm, vorzugsweise von 0,3 bis 1,5 μm und werden durch Filtrieren, Zentrifugieren oder Dekandieren von der Mutterlauge abgetrennt und mit einer geeigneten Waschflüssigkeit, vorzugsweise Wasser, gewaschen. Anschließend werden die Kristalle ggf. getrocknet und zur Entfernung des Templats bei einer Temperatur zwischen 400°C und 1.000°C, vorzugsweise zwischen 500°C und 750°C calciniert.

Gemäß einer bevorzugten Ausführungsform der vorliegenden Erfindung wird die Kristallsuspension nach dem Hydrothermalschritt und vor der Abtrennung der Kristalle neutralisiert. Die Kristallsuspension, wie sie nach Beendigung der Kristallisation bei dem erfindungsgemäßen Verfahren anfällt, ist aufgrund des Überschusses an basischem quartären Ammoniumsalz alkalisch und weist in der Regel einen pH von größer 12 auf. Wird der pH-Wert der Suspension auf einen Wert von 7 bis 10, vorzugsweise 7 bis 8.5 herabgesetzt, beobachtet man eine stärkere Agglomeration der Primärkristallite. Dadurch wird die Filtrierbarkeit der Suspension stark verbessert, so daß die Abtrennung mit standardmäßigen Membranfiltern ohne Durchschlagen des Produktes und mit üblichen Filtrationszeiten durchgeführt werden kann. Somit kann mit dieser bevorzugten Ausführungsform die Effizienz des erfindungsgemäßen Verfahrens noch weiter gesteigert werden.

Die Herabsetzung des pH-Werts der Kristallsuspension kann entweder durch Zugabe von Säure wie z.B. Mineralsäuren oder organischen Säuren nach Beendigung des Hydrothermalschritts oder durch Kristallisation bei erhöhten Temperaturen, z.B. bei 200°C bis 220°C erfolgen. Im letzteren Falle wird die quartäre Ammoniumverbindung z.B. Tetra-n-propylammoniumhydroxid unter Verbrauch von Hydroxidionen thermisch zersetzt. Bevorzugte Säuren sind Salzsäure und Essigsäure.

Durch den niedrigeren pH-Wert der Kristallsuspension fallen die darin gelösten Silikate und Titanate zumindest teilweise aus, so daß der abgetrennte titanhaltige Zeolith geringe Menge an Titanverbindungen enthält, die nicht ins Gitter eingebaut sind. Einen negativen Einfluß auf die Aktivität des resultierenden Katalysators wird allerdings nicht beobachtet.

Die erfindungsgemäßen kristallinen titanhaltigen Zeolithe werden in Pulverform erhalten. Für ihre Anwendung als Oxidationskatalysator können sie wahlweise durch bekannte Verfahren zur Verformung von Pulverkatalysatoren wie beispielsweise Granulation, Sprühtrocknen, Sprühgranulation oder Extrusion in eine zur Anwendung geeignete Form wie beispielsweise Microgranulate, Kugeln, Tabletten, Vollzylinder, Hohlzylinder oder Wabenkörper gebracht werden.

Der erfindungsgemäß hergestellte titanhaltige Zeolith kann als Katalysator bei Oxidationsreaktionen mit $H_2O_2$ eingesetzt werden. Insbesondere kann der erfindungsgemäße titanhaltige Zeolith als Katalysator bei der Epoxidierung von Olefinen mittels wäßrigem Wasserstoffperoxid in einem mit Wasser mischbaren Lösungsmittel verwendet werden.

Das erfindungsgemäße Verfahren hat den Vorteil, daß aufgrund des niedrigen Verhältnisses von Ammonium zu Silicium, bezogen auf die produzierte Zeolithmenge im Vergleich zum Stand der Technik, eine wesentlich geringere Menge an quartärer Ammoniumverbindung eingesetzt werden muß, die die teuerste der verwendeten Ausgangsverbindungen ist. Somit konnte die Wirtschaftlichkeit des Herstellungsverfahrens deutlich verbessert werden. Darüber hinaus kann die Ausbeute an Produkt, bezogen auf die Masse des Synthesegels gesteigert werden kann, wenn das molare Verhältnis von $H_2O$ zu Si, bezogen auf die Ausgangsverbindungen, in dem angegebenen Bereich gehalten werden. Überraschenderweise wurde festgestellt, daß durch diese Maßnahmen zur Optimierung der Verfahrensökonomie sich im Gegensatz zur Lehre des Standes der Technik die Katalysatoraktivität nicht verschlechtert, sondern im Gegenteil ein neues Produkt erhalten wird, das sich durch eine verbesserte Aktivität trotz einer im Vergleich zum Stand der Technik größeren Primärkristallitgröße auszeichnet.

Die Primärkristallitgröße des titanhaltigen Zeolithen, der nach dem erfindungsgemäßen Verfahren hergestellt wird, liegt im Bereich zwischen 0,2 bis 2,0 μm, vorzugsweise zwischen 0,3 bis 1,5 μm, während nach dem Stand der Technik eine Kristallitgröße im Bereich zwischen 0,1 bis 0,2 μm als optimal für die Katalysatoraktivität beschrieben wird.

Die vorliegende Erfindung soll nun anhand der Beispiele genauer erläutert werden:

Vergleichsbeispiel 1

Herstellung von EUROTS-1

EUROTS-1 wurde in Anlehnung an die Arbeitsvorschrift in Martens et al., "Applied Catalysis A: General, 99 (1993) 71-84" hergestellt. In einem mit Stickstoff inertisierten 10-l-Autoklaven wurden Tetraethylorthosilikat vorgelegt und unter Rühren Tetraethylorthotitanat zugegeben und nach Beendigung der Zugabe die resultierende Mischung auf ca. 1,0°C abgekühlt. Anschließend wurde bei dieser Temperatur unter Rühren innerhalb von etwa fünf Stunden eine 40 Gew.-%-ige Lösung von Tetra-n-propylammoniumhydroxid und entionisiertes Wasser mittels einer Schlauchpumpe zudosiert. Die Mengen wurden dabei so gewählt, daß das molare Verhältnis von $SiO_2$ zu $TiO_2$ gleich 35 ist, das molare Verhältnis von N zu Si gleich 0,36 beträgt und das molare Verhältnis von $H_2O$ zu $SiO_2$ gleich 28,2 beträgt. Die Reaktionsmischung wurde aber zunächst milchigtrüb, die gebildeten Feststoffe gingen bei weiterer Zugabe von Tetra-n-propylammoniumhydroxid jedoch vollständig wieder in Lösung.

Um die Hydrolyse zu vervollständigen und das gebildete Ethanol abzudestillieren, wurde die Reaktionsmischung zunächst auf ca. 80°C und innerhalb von etwa drei Stunden auf max. 95°C erhitzt. Das dabei abdestillierte Ethanol-Wasser-Azeotrop wurde durch das gleiche Volumen doppelt entionisiertes Wasser ersetzt. Anschließend wurde das Synthesesol auf 175°C erhitzt und bei dieser Temperatur 12 Stunden lang gehalten. Nach Abkühlen der resultierenden Titansilicalitsuspension wurde der gebildete Feststoff durch Zentrifugieren von der stark basischen noch tetra-n-propylammoniumhydroxidhaltigen Mutterlauge abgetrennt, gewaschen und über Nacht bei 120°C getrocknet und schließlich in Luftatmosphäre bei 550°C fünf Stunden in einem Muffelofen calciniert.

Hieraus ergibt sich ein Verbrauch von 2,9 kg 40 gewichtsprozentiger Tetra-n-propylammoniumhydroxidlösung pro kg TS-1 und eine Ausbeute von 50 g TS-1 pro kg Synthesegel bei einer Aktivitätskennzahl von 22 min[-1].

Die Aktivitätskennzahl wurde wie folgt bestimmt:

In einem thermostatisierten Laborautoklav mit Begasungsrührer wurden dann 1,0 g des im Vergleichsbeispiel 1 hergestellten Titansilicalitkatalysators in 300 ml Methanol bei 40°C unter Propylenatmosphäre vorgelegt und das Lösungsmittel bei 3 bar Überdruck mit Propylen gesättigt. Dann werden unter Rühren 13,1 g 30 Gew.-% wäßrige Wasserstoffperoxidlösung in einer Portion zugegeben und die Reaktionsmischung bei 40°C und 3 bar gehalten, wobei über einen Druckregler Propylen nachdosiert wurde, um den Verbrauch durch die Reaktion auszugleichen. In regelmäßigen Abständen wurden über ein Filter Proben entnommen und der Wasserstoffperoxidgehalt der Reaktionsmischung durch Redoxtitration mit Cer(IV)sulfat-Lösung bestimmt. Die Auftragung von $1n(c/c_0)$ gegen die Zeit t, wobei c die gemessene $H_2O_2$-Konzentration zum Zeitpunkt t und $c_0$ die berechnete $H_2O_2$-Konzentration zu Beginn der Reaktion ist, ergibt eine Gerade. Aus der Steigerung der Geraden wurde mit der Beziehung $\frac{dc}{dt} = k.c.c_{kat}$, worin $c_{kat}$ für die

Katalysatorkonzentration in kg Katalysator je kg Reaktionsmischung steht, die Aktivitätskennzahl bestimmt.

Beispiel 1

Herstellung eines Titansilicalit 1 gemäß der vorliegenden Erfindung.

Die Vorgehensweise wie für Vergleichsbeispiel 1 beschrieben wurde wiederholt mit der Ausnahme, daß solche Mengen an Ausgangsverbindungen eingesetzt wurden, daß sich ein molares Verhältnis, bezogen auf die Ausgangsverbindungen von N zu Si von 0,17 und von $H_2O$ zu Si von 13,3 ergab. Das Molverhältnis von Si zu Ti von 35 wurde beibehalten.

Bei diesem Syntheseverfahren ergab sich ein Verbrauch an Tetra-n-propylammoniumhydroxid (40 %-ige Lösung) von 1,6 kg pro kg TS-1, eine Ausbeute von 110 g TS-1 pro kg Synthesegel bei einer Aktivitätskennzahl des resultierenden Katalysators 31,6 min[-1] nach 12-stündigem Kristallisieren.

Dieser Vergleich zeigt bereits, daß mit einem Verfahren, das 50 % weniger teures Tetra-n-propylammoniumhydroxid verbraucht, die Ausbeute an Katalysator pro kg Synthesegel mehr als verdoppelt werden kann und trotzdem und entgegen den Erwartungen des Standes der Technik eine beträchtliche Steigerung der katalytischen Aktivität des resultierenden Katalysators erreicht werden kann.

Beispiele 2 und 3

Beispiel 1 wurde mit den in der Tabelle 1 angegebenen Verhältnissen der Einsatzstoffe wiederholt. Die resultierenden Produkteigenschaften sind ebenfalls in Tabelle 1 zusammengefaßt.

Vergleichsbeispiele 2 bis 4

Vergleichsversuch 1 wurde mit den in Tabelle 1 angegebenen Verhältnissen der Einsatzstoffe wiederholt. Die Produkteigenschaften der resultierenden Titansilicalit-1-Proben sind ebenfalls in Tabelle 1 zusammengefaßt.
Das Molverhältnis Si zu Ti bezogen auf die Ausgangsverbindungen wurde bei allen Versuchen konstant bei 35 gehalten.

Tabelle 1

| Beispiel | Einsatzstoffverhältnis | | Produkteigenschaften Titansilicalit-1 | | |
| | $H_2O/Si$ (mol/mol) | N/Si (mol/mol) | Gew.-% $TiO_2$ | Kristallitgröße ($\mu$m) | katalyt. Aktivität ($min^{-1}$) |
|---|---|---|---|---|---|
| V1 | 28,2 | 0,36 | 2,4 | 0,1-0,2 | 22,1 |
| V2 | 16,8 | 0,10 | 3,0 | 1,0-4,0 | 22,1 |
| V3 | 16,8 | 0,08 | 2,8 | 2-3 | 25,2 |
| V4 | 9,3 | 0,22 | 2,8 | 0,2 | 22,2 |
| | | | | | |
| 1 | 13,3 | 0,17 | 3,2 | 0,4 | 31,6 |
| 2 | 16,9 | 0,12 | 3,2 | 0,5-1,5 | 34,4 |
| 3 | 16,8 | 0,15 | 3,3 | 0,5 | 32,1 |

Der Vergleich zwischen den Beispielen gemäß der vorliegenden Erfindung und den Vergleichsbeispielen zeigt, daß die Korrelation zwischen Verhältnis von Stickstoff zu Silicium in den Ausgangsverbindungen und Primärkristallitgröße den im Stand der Technik beschriebenen Verlauf wiedergibt. Die Versuche zeigen, daß eine optimale Primärkristallitgröße zwischen 0,1 und 0,2 $\mu$m bei einem Verhältnis von N zu Si von 28 oder größer erreicht wird.
Aber im Gegensatz zu den Prognosen aus dem Stand der Technik führt ein verringertes Molverhältnis von N zu Si nicht zu einer Abnahme der katalytischen Aktivität, im Gegenteil, die Zunahme des Einbaus an Titan in das Kristallgitter bei den Beispielen gemäß der vorliegenden Erfindung, überkompensiert den negativen Einfluß der zunehmenden Primärkristallitgrößen auf die Katalysatoraktivität.
Der Vergleich mit der Synthese des EUROTS-1-Katalysators (Vergleichsbeispiel 1) ergibt, daß nach dem erfindungsgemäßen Verfahren ein Katalysator mit erhöhter Aktivität hergestellt werden kann, wobei das Verfahren im Vergleich zum Stand der Technik zu einem deutlich verringerten Verbrauch an teuren Ausgangsmaterialien wie Tetran-propylammoniumhydroxid und zu einer deutlich gesteigerten Ausbeute an Titansilicalit pro kg Synthesegel führt und somit noch zusätzlich die Wirtschaftlichkeit des Syntheseverfahrens verbessert werden konnte.

Beispiel 4

Beispiel 1 wurde wiederholt, wobei die Titansilicalitkristalle aber nicht durch Zentrifugation sondern durch Filtration abgetrennt wurden. Die basische (pH = 12,5) Kristallsuspension wurde auf eine Filternutsche mit Blaubandfilter gegeben. Dabei blieb kein Feststoff auf der Nutsche zurück.

Beispiele 5 und 6

Beispiel 4 wurde wiederholt, wobei der pH-Wert der Kristallsuspension vor der Filtration durch Zugabe von Salzsäure auf den in Tabelle 2 angegebenen Wert reduziert wurde. In beiden Fällen konnte der Titansilicalit ohne Durchschlagen des Produkts mit einer Filternutsche mit Blaubandfilter abgetrennt werden.
Der Titansilicalit wurde wie in Vergleichsbeispiel 1 gewaschen, über Nacht bei 120°C getrocknet und in Luftatmosphäre bei 550°C fünf Stunden in einem Muffelofen calciniert. Danach wurde das Produkt auf Titangehalt, qualitativ auf Nichtgitter-Titanverbindungen und auf Aktivität wie oben angegeben untersucht. Die Ergebnisse sind ebenfalls in Tabelle 2 angegeben.

Tabelle 2

| Beispiel | | Produkteigenschaften | | |
|---|---|---|---|---|
| | pH-Wert Suspension | Titangehalt (Gew.-% $TiO_2$) | Gehalt an Nichtgitter-Ti | Aktivitätskennzahl $(min^{-1})$ |
| 5 | 8,5 | 3,2 | + | 29,2 |
| 6 | 7 | 3,5 | +++ | 30,9 |

Die qualitative Abschätzung des Gehalts an Nichtgitter-Titanverbindungen erfolgte anhand der Intensität der DR-UV-Vis-Banden zwischen 250 und 300 nm. Das Produkt des Beispiels 6 hatte einen höheren Gehalt an Nichtgitter-Titanverbindungen als das Produkt des Beispiels 5. Wie der Vergleich der Aktivitätskennzahlen von Beispiel 5 und 6 mit Beispiel 1 zeigt, hat die Absenkung des pH-Werts der Kristallsuspension einen zu vernachlässigenden Einfluß auf die Aktivität des Katalysators.

**Patentansprüche**

1. Verfahren zur Herstellung eines titanhaltigen Zeolithen, wobei ein Synthesegel durch Zusammengeben und Hydrolisieren einer hydrolisierbaren Siliciumverbindung, einer hydrolisierbaren Titanverbindung und einer basischen quartären Ammoniumverbindung in wäßrigem Medium in Mengen, so daß sich bezogen auf die Ausgangsverbindungen ein molares Verhältnis von Si/Ti von größer gleich 30 und von N/Si von 0,12 bis kleiner 0,20 ergibt, ausgebildet wird und anschließend das Synthesegels bei einer Temperatur von 150°C bis 220°C für eine Zeitdauer von weniger als 3 Tage kristallisiert wird.

2. Verfahren nach Anspruch 1,
   wobei das molare Verhältnis von N/Si 0,12 bis 0,17 beträgt.

3. Verfahren nach einem der vorstehenden Ansprüche,
   wobei das molare Verhältnis von $H_2O$/Si bezogen auf die Ausgangsverbindungen 10 bis 20 beträgt.

4. Verfahren nach Anspruch 3,
   wobei das molare Verhältnis von $H_2O$/Si 12 bis 17 beträgt.

5. Verfahren nach einem der vorstehenden Ansprüche,
   wobei die hydrolisierbare Siliciumverbindung ein Tetraalkylorthosilikat, vorzugsweise Tetraethylorthosilikat ist und die hydrolisierbare Titanverbindung ein Tetraalkylorthotitanat, vorzugsweise Tetraethylorthotitanat ist.

6. Verfahren nach Anspruch 5,
   wobei die Hydrolyse der hydrolisierbaren Titan- und Siliciumverbindungen durch Abdestillieren der entstehenden Alkohole unterstützt wird und fakultativ das Volumen des abdestillierten Alkohols zumindest teilweise durch Zugabe von Wasser zur Reaktionsmischung ersetzt wird.

7. Verfahren nach einem der vorstehenden Ansprüche,
   wobei die quartäre Ammoniumverbindung ein Tetraalkylammoniumhydroxid, vorzugsweise Tetra-n-propylammoniumhydroxid ist.

8. Verfahren nach einem der vorstehenden Ansprüche,
   wobei der pH-Wert der Kristallsuspension aus dem Kristallisationsschritt auf einen Wert von 7 bis 10, vorzugsweise von 7 bis 8,5 eingestellt wird

9. Verfahren nach einem der vorstehenden Ansprüche,
   wobei der titanhaltige Zeolith abgetrennt, getrocknet und calciniert wird.

10. Titanhaltiger Zeolith erhältlich durch ein Verfahren nach einem der vorstehenden Ansprüche mit einer Primärkristallitgröße von 0,2 bis 2,0 µm.

11. Verfahren zur Epoxidierung von Olefinen durch Umsetzen von Olefin mit wäßrigem Wasserstoffperoxid in einem mit Wasser mischbaren Lösungsmittel in Gegenwart eines titanhaltigen Zeolithen als Katalysator, der durch das Verfahren nach Anspruch 9 erhältlich ist.

12. Verwendung eines titanhaltigen Zeolithen, der nach dem Verfahren nach Anspruch 8 erhältlich ist, als Katalysator für die Epoxidierung von Olefinen mit Wasserstoffperoxid.

**Europäisches Patentamt**

# EUROPÄISCHER RECHERCHENBERICHT

Nummer der Anmeldung

EP 00 10 4324

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | THANGARAJ A ET AL: "STUDIES ON THE SYNTHESIS OF TITANIUM SILICALITE, TS-1" ZEOLITES,US,BUTTERWORTH-HEINEMANN, Bd. 12, Nr. 8, 1. November 1992 (1992-11-01), Seiten 943-950, XP000198990 ISSN: 0144-2449 * das ganze Dokument * | 1,2,5,7, 9,11,12 | C01B37/00 B01J29/04 C07D301/12 |
| A | | 3,4,6,8, 10 | |
| A | DE 196 23 972 A (DEGUSSA) 18. Dezember 1997 (1997-12-18) * Ansprüche 1,7,8 * * Seite 3, Zeile 17 - Seite 4, Zeile 29 * | 1-4,7,9, 11,12 | |
| A | BE 1 001 038 A (ENIRICERCHE S P A ENICHEM SYNT) 20. Juni 1989 (1989-06-20) * Beispiele 1,3 * * Seite 3, Zeile 1 - Seite 4, Zeile 9 * | 1-10 | |
| A | ZHANG G ET AL: "PREPARATION OF COLLOIDAL SUSPENSIONS OF DISCRETE TS-1 CRYSTALS" CHEMISTRY OF MATERIALS,US,AMERICAN CHEMICAL SOCIETY, WASHINGTON, Bd. 9, Nr. 1, 1997, Seiten 210-217, XP000683976 ISSN: 0897-4756 * das ganze Dokument * | 1,5,9,10 | RECHERCHIERTE SACHGEBIETE (Int.Cl.7)  C01B |
| A | FR 2 471 950 A (SNAM PROGETTI) 26. Juni 1981 (1981-06-26) * das ganze Dokument * | 1,3,4,6, 9 | |
| D,A | & US 4 410 501 A 18. Oktober 1983 (1983-10-18) | | |

-/--

Der vorliegende Recherchenbericht wurde für alle Patentansprüche erstellt

| Recherchenort | Abschlußdatum der Recherche | Prüfer |
|---|---|---|
| DEN HAAG | 21. Juli 2000 | Rigondaud, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................
& : Mitglied der gleichen Patentfamilie, übereinstimmendes Dokument

EPO FORM 1503 03.82 (P04C03)

**Europäisches Patentamt**

## EUROPÄISCHER RECHERCHENBERICHT

Nummer der Anmeldung

EP 00 10 4324

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | A. THANGARAJ ET AL.: "CATALYTIC PROPERTIES OF CRYSTALLINE TITANIUM SILICALITES." JOURNAL OF CATALYSIS., Bd. 130, 1991, Seiten 1-8, XP002143172 MN US ----- | | |
| | | | **RECHERCHIERTE SACHGEBIETE (Int.Cl.7)** |

Der vorliegende Recherchenbericht wurde für alle Patentansprüche erstellt

| Recherchenort | Abschlußdatum der Recherche | Prüfer |
|---|---|---|
| DEN HAAG | 21. Juli 2000 | Rigondaud, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
....................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 00 10 4324

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-07-2000

| Im Recherchenbericht angeführtes Patentdokument | Datum der Veröffentlichung | Mitglied(er) der Patentfamilie | Datum der Veröffentlichung |
|---|---|---|---|
| DE 19623972 A | 18-12-1997 | KEINE | |
| BE 1001038 A | 20-06-1989 | IT 1216500 B | 08-03-1990 |
| FR 2471950 A | 26-06-1981 | IT 1127311 B | 21-05-1986 |
| | | AR 231610 A | 31-01-1985 |
| | | AT 385022 B | 10-02-1988 |
| | | AT 586880 A | 15-07-1987 |
| | | AU 537263 B | 14-06-1984 |
| | | AU 6451380 A | 02-07-1981 |
| | | BE 886812 A | 22-06-1981 |
| | | BG 39637 A | 15-07-1986 |
| | | BR 8008379 A | 07-07-1981 |
| | | CA 1155830 A | 25-10-1983 |
| | | CH 645599 A | 15-10-1984 |
| | | CS 257253 B | 15-04-1988 |
| | | DD 155420 A | 09-06-1982 |
| | | DE 3047798 A | 22-10-1981 |
| | | DK 493980 A,B, | 22-06-1981 |
| | | ES 498480 D | 16-11-1981 |
| | | ES 8200823 A | 16-02-1982 |
| | | GB 2071071 A,B | 16-09-1981 |
| | | GR 72832 A | 07-12-1983 |
| | | HU 183272 B | 28-04-1984 |
| | | IL 61654 A | 30-03-1984 |
| | | IN 154032 A | 08-09-1984 |
| | | JP 1042889 B | 18-09-1989 |
| | | JP 1561240 C | 31-05-1990 |
| | | JP 56096720 A | 05-08-1981 |
| | | LU 83014 A | 23-07-1981 |
| | | NL 8006939 A,B, | 16-07-1981 |
| | | NO 803859 A,B, | 22-06-1981 |
| | | PH 17732 A | 21-11-1984 |
| | | PL 228600 A | 07-08-1981 |
| | | PT 72248 A,B | 01-01-1981 |
| | | RO 81245 A | 01-06-1983 |
| | | SE 447818 B | 15-12-1986 |
| | | SE 8008961 A | 22-06-1981 |
| | | RU 2076775 C | 10-04-1997 |
| | | TR 21473 A | 04-07-1984 |
| | | US 4410501 A | 18-10-1983 |
| | | YU 316780 A | 30-09-1983 |
| | | ZA 8007233 A | 27-01-1982 |
| | | ZM 11280 A | 21-12-1981 |

EPO FORM P0461

Für nähere Einzelheiten zu diesem Anhang : siehe Amtsblatt des Europäischen Patentamts, Nr. 12/82